# EUROPEAN PATENT APPLICATION

(11) **EP 1 920 817 A1**
(43) Date of publication of application: **14.05.2008**
(21) Application number: 07119698.4
(22) Date of filing: 31.10.2007
(51) Int. Cl.: B01D 53/04

(54) **Oxygen delivery system**

(30) Priority: 10.11.2006 US 595375
(71) Applicant: Delphi Technologies, Inc., Troy, Michigan 48007 (US)
(72) Inventor: Chekal, Michael P., Brighton, MI 48116 (US); Pelletier, Dana G., Ortonville, MI 48462 (US); Mc Clain, Michael S., Ortonville, MI 48462 (US)
(74) Representative: Denton, Michael John

(57) **Abstract**

An oxygen delivery system includes first and second sieve beds configured to selectively receive a supply fluid and separate a substantially oxygen-rich fluid therefrom. A patient conduit having a patient outlet is in alternate selective fluid communication with the sieve beds. The system includes a breath-detection device in fluid communication with the patient conduit, configured to calculate a rate of breathing and to trigger, in response to a breath detection, an output of a predetermined volume of the substantially oxygen-rich fluid alternately from a respective one of the sieve beds. The system also includes a device configured to measure the output flow rate at predetermined intervals. The output, having a target flow rate and a flow rate, occurs during a respective dynamically adjusted patient oxygen delivery phase, which is dependent upon the target flow rate, the output flow rate, and/or the breathing rate.

## Description

### Technical Field:

The present disclosure relates generally to oxygen delivery, and more particularly to a system and method for optimizing the delivery of oxygen having a desired level of purity.

### Background of the invention:

Prior oxygen delivery systems have been configured to deliver oxygen at a constant flow rate or in pulses having a fixed duration and/or fixed valve timing based upon a predetermined flow setting.

Constant flow oxygen delivery systems provide output during periods of patient inhalation, exhalation, and therebetween. It is recognized that substantially oxygen-rich fluid provided at times other than during patient inhalation is generally inefficient, and, in some instances, may waste the oxygen-rich fluid. Additionally, due to the constant operation of constant flow oxygen delivery systems, power consumption may be higher than necessary; and if battery-operated, this may result in reduced battery life of the oxygen delivery system.

Previous oxygen delivery systems configured to output fixed-duration and/or fixed-timing pulses of substantially oxygen-rich fluid have been regulated by a flow setting, which may be manually input. Such fixed-duration and/or fixed-timing systems may suffer from shortcomings similar to those of the constant flow oxygen delivery systems. More specifically, the pulses are output at predetermined intervals and/or for a predetermined duration. Thus, the pulses of substantially oxygen-rich fluid may not be delivered during patient inhalation, when the fluid may be optimally received and utilized by the patient. As such, oxygen delivery systems that output fixed-duration and/or fixed-timing pulses also may be relatively inefficient with respect to the amount of oxygen inhaled by the patient as compared to the amount of substantially oxygen-rich fluid generated and output.

As such, it would be desirable to provide an improved system and method for optimizing the delivery of substantially oxygen-rich fluid.

### Summary of the invention:

An oxygen delivery system includes a supply fluid containing oxygen. The system further includes first and second sieve beds, each sieve bed in selective fluid communication with the supply fluid and configured to selectively receive the supply fluid during a predetermined supply period. Each of the first and second sieve beds is further configured to separate a substantially oxygen-rich fluid from the supply fluid. A patient conduit having a patient outlet is in alternate selective fluid communication with the first and second sieve beds. The oxygen delivery system includes a breath-detection device in fluid communication with the patient conduit, which breath detection device may be configured to measure a rate of breathing. The breath-detection device is configured to trigger an output, in response to a breath detection, of a predetermined volume of the substantially oxygen-rich fluid alternately from a respective one of the first sieve bed or the second sieve bed, the output having a target flow rate and a flow rate. The oxygen delivery system also includes a device in fluid communication with patient conduit, the device configured to measure the output flow rate at predetermined intervals. The predetermined volume is output via the patient outlet during a respective dynamically adjusted patient oxygen delivery phase. The dynamically adjusted patient oxygen delivery phase is dependent upon the target flow rate, the output flow rate, and/or the breathing rate.

### Brief Description of the Drawings:

Features and advantages of the present disclosure will become apparent by reference to the following detailed description and drawings, in which like reference numerals correspond to similar, though not necessarily identical components. For the sake of brevity, reference numerals or features having a previously described function may not necessarily be described in connection with other drawings in which they appear.
Figure 1 is a schematic diagram of an embodiment of an oxygen delivery system;
Figure 2 is a schematic diagram of another embodiment of an oxygen delivery system;
Figure 3 is a flow diagram depicting an embodiment of a method for delivering oxygen; and
Figure 4 is a state diagram depicting an embodiment of a method of delivering a substantially oxygen-rich fluid from an oxygen delivery system.

### Description of the Preferred Embodiments:

Embodiment(s) of the oxygen delivery system disclosed herein advantageously provide oxygen to a patient during a dynamically adjusted oxygen delivery phase. A supply fluid is selectively provided alternately to two sieve beds, which are configured to separate a substantially oxygen-rich fluid from the supply fluid. The sieve beds may alternately provide the substantially oxygen-rich fluid to a patient in response to a breath inhalation. As such, embodiment(s) of the hereindescribed oxygen delivery system may provide a patient with a substantially pulse of oxygen of a desired purity, and/or may substantially prevent the delivery of a pulse having a relatively low oxygen level, while optimizing timing to allow synchronization with a patient breathing rate, including a substantially fast breathing rate.

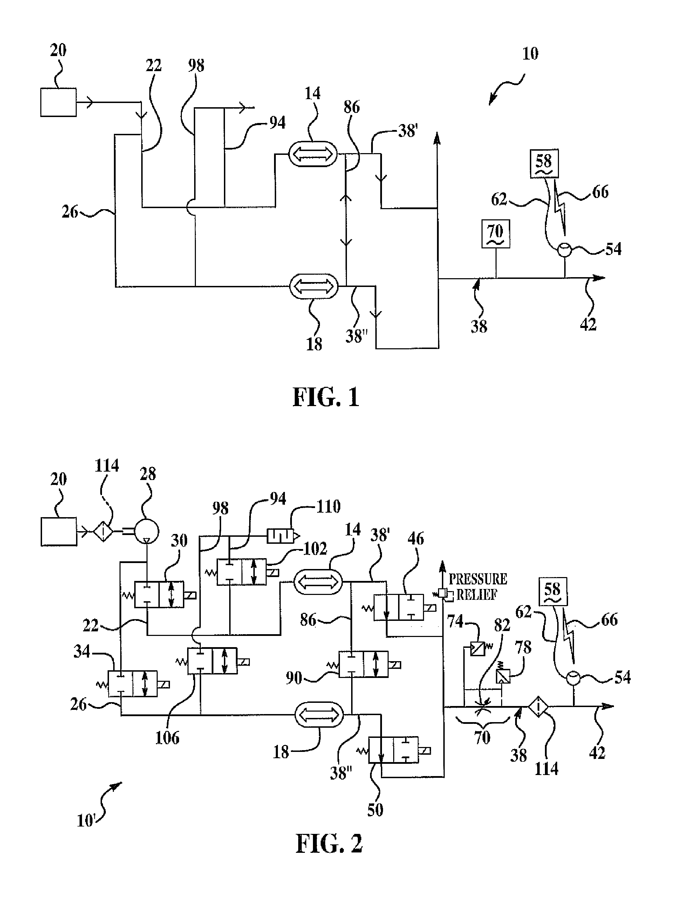

Referring to Figs. 1 and 2, embodiments of an oxygen delivery system 10, 10' are depicted having first 14 and second 18 purifying systems, each in selective fluid communication with a supply fluid containing oxygen. The supply fluid may come from any suitable supply source 20. In an embodiment, each of the first 14 and second 18 purifying systems are configured to selectively receive the supply fluid during a predetermined supply period. The first 14 and second 18 purifying systems may receive the supply fluid via first 22 and second 26 supply conduits, respectively.

As depicted in Fig. 2, the supply fluid may be compressed, if desired. It is to be understood that the compression may be achieved by any suitable means, e.g. via a compressor 28. Further, the compressor 28 may be any suitable compressor. In an embodiment, compressor 28 is a scroll compressor. As such, the supply fluid may have any suitable pressure, as desired or required.

Additionally, the first 22 and second 26 supply conduits may be configured with first 30 and second 34 supply valves, respectively. It is to be understood that when one of the first 14 or second 18 purifying systems are receiving the supply fluid, the respective one of the first 30 or second 34 supply valves is in an open position. In an embodiment, the supply valves 30, 34 are configured as two-way supply valves.

The first 30 and second 34 supply valves are configured to direct the supply fluid to a respective one of the first 14 or second 18 purifying systems at a particular time. As such, in an embodiment, when the supply fluid is directed to one of the first 14 or second 18 purifying systems, the supply fluid is prevented from flowing to the other of the second 18 or the first 14 purifying systems. It is contemplated, however, that in addition to the first 30 and second 34 supply valves preventing the supply fluid from flowing to both the first 14 and second 18 purifying systems at the same time, the first 30 and second 34 supply valves may also prevent the supply fluid from flowing to either of the first purifying system 14 or the second purifying system 18 at a particular time, as desired.

Referring again to Figs. 1 and 2 together, after receiving the supply fluid, the first 14 and second 18 purifying systems are each configured to separate a substantially oxygen-rich fluid from the supply fluid. In an embodiment, first 14 and second 18 purifying systems are each sieve beds 14, 18, respectively, and are configured to adsorb an oxygen-depleted fluid from the supply fluid during the separating. As such, the first and second sieve beds 14, 18 may substantially separate an oxygen-rich fluid from a substantially oxygen-depleted fluid. It is to be understood that the sieve bed 14, 18 is one embodiment of a purifying system 14, 18. Further, "purifying system" is intended to be interpreted broadly to include all suitable purifying systems, with or without sieve beds, and, if sieve beds are used, to include any suitable type and/or configuration of sieve bed. Standard Pressure Swing Adsorption (PSA) is one technology used to separate oxygen in which any number of sieve columns in a single purifying system/sieve bed 14, 18 may be used. Generally, two columns of sieve are used in a single sieve bed 14, 18, but it is to be understood that the present disclosure is not intended to be limited to single or multiple columns of sieve, but rather any suitable number of sieves in a respective sieve bed 14, 18 to optimize a desired size and/or output flow requirements. Other examples of oxygen separation technologies suitable for purifying system 14, 18 include, but are not limited to chemical separation techniques, electrically charged particle separation to extract the oxygen, and/or the like, and/or combinations thereof.

In an embodiment, the first and second sieve beds 14, 18 are configured to separate the fluid by utilizing pressure swing adsorption (PSA). As such, in an embodiment, the supply fluid is air, and the first and second sieve beds 14, 18 are each configured to substantially adsorb at least nitrogen out of the air to substantially separate the oxygen in the air from at least the nitrogen.

As used herein, substantially oxygen-rich fluid is to be understood to include a fluid comprising a majority of breathable oxygen. In an embodiment, the substantially oxygen-rich fluid is a gas. As a non-limiting example, the substantially oxygen-rich fluid is a gas containing from about 70% oxygen by volume to about 100% oxygen by volume. In an alternate embodiment, the substantially oxygen-rich fluid is a gas containing from about 82% oxygen by volume to about 98% oxygen by volume. In yet a further alternate embodiment, the substantially oxygen-rich fluid contains at least about 87% oxygen by volume.

A patient conduit 38 having a patient outlet 42 is in alternate selective fluid communication with the first and second sieve beds 14, 18. As an example, the patient conduit 38 may be formed at least partially from flexible plastic tubing. In an embodiment, the patient conduit 38 is configured substantially in a "Y" shape. As such, the patient conduit 38 may have a first conduit portion 38' and a second conduit portion 38", which are in communication with the first sieve bed 14 and second sieve bed 18, respectively, and merge together before reaching the patient outlet 42, as depicted in Figs. 1 and 2. The patient outlet 42 may be any orifice in the patient conduit 38 configured to output the substantially oxygen-rich fluid for patient use. The patient outlet 42 may additionally be configured with a nasal cannula, a respiratory mask, or any other suitable device, as desired or required.

As depicted in Fig. 2, the first conduit portion 38' and the second conduit portion 38" may be configured with a first patient valve 46 and a second patient valve 50, respectively. In an embodiment, the first 46 and second 50 patient valves are configured as two-way valves. It is contemplated that when the substantially oxygen-rich fluid is delivered from one of the first or second sieve beds 14, 18 to the patient conduit 38, the respective one of the first 46 or second 50 patient valves is open. Further, when the respective one of the first 46 or second 50 patient valves is open, the respective one of the first 30 or second 34 supply valves is closed. Yet further, when one of the first 46 or second 50 patient valves is open, both of the first 30 and second 34 supply valves may be closed.

Referring yet again to Figs. 1 and 2 together, in an embodiment, a breath-detection device 54 is in fluid communication with the patient conduit 38. The breath-detection device 54 may be configured to trigger an output of a predetermined volume of the substantially oxygen-rich fluid from a respective one of the first sieve bed 14 or the second sieve bed 18 in response to a breath inhalation detected by the breath-detection device 54. Alternately or additionally, a separate control device connected to the breath-detection device 54 may be configured to trigger the output of the predetermined volume in response to the breath inhalation detection. The output has a target flow rate and (an actual) flow rate. In an embodiment, the target flow rate is a predetermined value manually input by a user/operator. The predetermined volume is output via the patient conduit 38 and the patient outlet 42 during a respective dynamically adjusted patient oxygen delivery phase.

In an embodiment, the duration of the dynamically adjusted patient oxygen delivery phase is dependent upon at least one of the output target flow rate, the output flow rate, the breathing rate, or combinations thereof. In an alternate embodiment, the duration of the dynamically adjusted patient oxygen delivery phase is dependent upon each of the output target flow rate, the output flow rate (e.g., as detected via flow-sensor feedback), and the breathing rate.

The duration of the dynamically adjusted patient oxygen delivery phase may be calculated, in one embodiment, taking into account the target flow rate. For each equivalent of Liter Per Minute (LPM) of flow of substantially oxygen-rich fluid desired, the system 10, 10' is configured to deliver a pulse of about 8.5 milliliters (mL). As such, if the system 10, 10' is set to deliver 2 LPMe of flow, each breath inhalation will trigger a pulse of the substantially oxygen-rich fluid that, when integrated, totals about 17 mL. The pulse size may be further calculated based upon the integration of the actual flow rate (LPM) versus time. This integration is performed by monitoring the output flow rate at predetermined intervals (e.g., every millisecond) and summing the monitored samples. The sum of the samples may be divided by the number of samples monitored per minute to convert the units to Liters. As such, in an embodiment, the pulse size is equal to the total of the sampled flow rates divided by (1000*60).

Since the separating/purifying process (e.g., a PSA process) requires a certain pressure for a fixed sieve bed size and target pulse size, the inlet pressure to the sieve beds 14, 18 is generally increased as breathing rate increases, and decreased as breathing rate decreases. For this reason, in embodiment(s) of the present disclosure, the speed of the motor (not shown) driving the air compressor 28 is advantageously, dynamically changed as needed to achieve the desired peak pressure inside each respective sieve bed 14, 18 to generate the target pulse size.

Following a dynamically adjusted patient oxygen delivery phase from the first 14 or second 18 sieve bed, breath detection may be masked for a predetermined masking time, for example, during the dynamically adjusted oxygen delivery phase and during a predetermined amount of time following the delivery phase. It is understood that such predetermined masking time may be configured to prevent the triggering of another dynamically adjusted patient oxygen delivery phase before sufficient substantially oxygen-rich fluid is available from the other of the second 18 or first 14 sieve bed. As used herein, sufficient substantially oxygen-rich fluid may be a pulse having a desired oxygen content. The predetermined masking time may be very short in duration. As a non-limiting example, the predetermined masking time may be about 500 milliseconds in length. In an embodiment, this masking time may also be dynamically adjusted based on the average breath rate. Further, in order to accommodate a maximum breathing rate of 30 Breaths Per Minute (BPM), a maximum mask time of 2 seconds may be used.

The breath-detection device 54 may include a pressure sensor configured to monitor the pressure of the substantially oxygen-rich fluid. More specifically, the pressure sensor may be configured to measure the pressure of the substantially oxygen-rich fluid in the patient conduit 38, for example, substantially near the patient outlet 42. Further, a system component, such as, for example, the breath-detection device 54 and/or a controller connected to the pressure sensor, may be configured to associate a predetermined drop in pressure of the substantially oxygen-rich fluid with a breath inhalation. A typical pressure drop may be, for example, less than 1 in. H₂O.

Further, the breath-detection device 54 and/or a suitable device connected thereto may be configured to calculate the rate of breathing based upon detected breath inhalations. The breathing rate may be detected based upon the monitoring of the pressure of the substantially oxygen-rich fluid in the patient conduit 38, particularly near the patient outlet 42. An exhalation generally follows each inhalation, and, thus, detection of each inhalation may be associated with a complete breath. As used herein, a rate of breathing may be defined as a number of breaths per minute, wherein one breath includes an inhalation and an exhalation.

The system 10, 10' may be configured to monitor the inhalations detected by the breath-detection device 54. The system 10, 10' may further include an alert system 58 configured to emit an alarm if the breath-detection device 54 fails to detect an inhalation within a predetermined amount of time. The alarm may be embodied as an aural alarm, a visual alarm, and/or a tactile alarm. As non-limiting examples, the predetermined amount of time may range from about 15 seconds to about 30 seconds.

The alert system 58 may be in operative communication with the breath-detection device 54 via a wired communication system 62 and/or a wireless communication system 66. As non-limiting examples, the wireless communication system 66 may utilize radio frequency (RF) communication and/or infrared communication.

In an embodiment, if an inhalation is not detected within a predetermined detection time (e.g., if the patient is disconnected from the system 10, 10'), the system 10, 10' may be configured to enter a suspend mode, which may be configured to reduce power consumption for example, by reducing motor speed in the compressor 28. As non-limiting examples, the predetermined detection time may be five minutes, ten minutes, fifteen minutes, or twenty minutes, etc. When a system 10, 10' enters suspend mode, substantially oxygen-rich fluid located in the first 14 or second 18 sieve beds may be held therein until a next inhalation is detected.

A device 70 may be in fluid communication with the patient conduit 38 and is configured to measure the output flow rate of the substantially oxygen-rich fluid. In an embodiment, the device 70 is configured to measure the output flow rate at predetermined intervals. Generally, the predetermined intervals may be dependent on various factors, e.g., the capabilities of the microprocessor (not shown) being used, the target accuracy of the measurement, and/or the like. In an example, the predetermined intervals may range from microseconds to about 50 milliseconds. As a non-limiting example, the predetermined intervals may be about every millisecond.

Referring again to Fig. 2, the device 70 may include a pressure sensor 74 and a differential sensor 78. The differential sensor 78 is configured to measure a change in the pressure across a metering orifice with laminar flow 82 located substantially in the patient conduit 38. A suitable temperature, such as room temperature, may be assumed; and pressure sensor 74 measures the gauge pressure. The two pressure values are used to determine the mass flow, from which the volume of the substantially oxygen-rich fluid may be calculated.

Referring back to Figs. 1 and 2, the first 14 and second 18 sieve beds may be configured, if desired, to transmit at least a portion of a remaining amount of the substantially oxygen-rich fluid to the other of the second sieve bed 18 or the first sieve bed 14 substantially after each respective dynamically adjusted oxygen delivery phase. The remaining amount of the fluid may be transmitted via the counterfill flow conduit 86.

The transmission of the remaining amount/portion thereof of the substantially oxygen-rich fluid from one of the sieve beds 14 to the other of the sieve beds substantially after a dynamically adjusted oxygen delivery phase may be referred to as "counterfilling." It is to be understood that the remaining amount may be any suitable amount of substantially oxygen-rich fluid remaining in the first 14 or second 18 sieve bed following the delivery of oxygen-rich fluid from the first 14 or second 18 sieve bed to the patient conduit 38. In an embodiment, the at least a portion of the remaining amount is substantially the entire amount of oxygen-rich fluid remaining in the first 14 or second 18 sieve bed; whereas, in an alternate embodiment, it is less than the entirety of the amount of oxygen-rich fluid remaining in the first 14 or second 18 sieve bed after the dynamically adjusted oxygen delivery phase.

In an embodiment, the duration of a counterfill may be dynamically adjusted. As such, a counterfill duration may be calculated for a flow setting and a predetermined breathing rate, e.g., an average breathing rate. As an example, the average breathing rate may be determined by calculating an average of the time duration between the prior five inhalations. In another example, the number of prior inhalations may be larger (for example, ten) or smaller (for example, three), as desired. In an embodiment, the counterfill duration ranges between about 400 milliseconds (mS) and about 800 mS. In an alternate embodiment, the counterfill duration is about 600 mS. A flow valve isolation time may be utilized substantially before and/or after the counterfill duration, as desired.

Counterfilling may be advantageous in that it hastens the availability of substantially oxygen-rich fluid (e.g., air having a desired level of oxygen content) from the respective sieve bed 14, 18 being counterfilled.

Referring again to Fig. 2, the counterfill flow conduit 86 may be configured with a counterfill flow valve 90. In an embodiment, the counterfill flow valve 90 is a two-way valve. The flow valve 90 is opened during the counterfilling of the respective first 14 and second 18 sieve beds. It is contemplated that when the counterfill flow valve 90 is open, all other valves in the system 10' are closed. The isolation time mentioned above may be used to substantially ensure that the opening of the counterfill valve 90 does not overlap the opening of other valves in the system 10, 10'.

Referring yet again to Figs. 1 and 2 together, in an embodiment, the first and second sieve beds 14, 18 are configured to purge the oxygen-depleted fluid (e.g., air with a relatively high nitrogen content and a relatively low oxygen content) remaining therein after the respective dynamically adjusted oxygen delivery phase (and after transmitting (counterfilling) the remaining amount of the substantially oxygen-rich fluid in the respective sieve bed 14, 18 to the other sieve bed 18, 14, if counterfilling is desirable). For example, the first and second sieve beds 14, 18 may purge substantially after delivering the substantially oxygen-rich fluid to the patient conduit 38 from the respective sieve bed 14, 18. In another embodiment, the respective sieve bed 14, 18 is configured to purge the remaining oxygen-depleted fluid substantially after counterfilling the other sieve bed 18, 14.

The first 14 and second 18 sieve beds may purge via the first purge conduit 94 and the second purge conduit 98, respectively. As used herein, "purging" is to be interpreted broadly to include purging substantially all of the oxygen-depleted fluid from the system 10,10', as well as purging a portion (i.e., less than all) of the oxygen-depleted fluid.

Referring again to Fig. 2, the first purge conduit 94 and the second purge conduit 98 may include a first purge valve 102 and a second purge valve 106, respectively. In an embodiment, the first purge valve 102 and second purge valve 106 are two-way valves. The first 102 or second 106 purge valves is open when the respective one of the first 14 or second 18 sieve beds is purging. In an embodiment, the first purge valve 102 or second 106 purge valve is open substantially while the other of the second sieve bed 18 or first 14 sieve bed is being supplied with fluid and is delivering the substantially oxygen-rich fluid. As such, the first 102 or second 106 purge valve may be open substantially while the respective others of the second supply valve 34 and second patient valve 50, or first supply valve 30 and first patient valve 46 are open. Additionally, if the system 10' has entered suspend mode (as described hereinabove), the respective first 102 or second 106 purge valve may be open, while all other system 10' valves may be closed.

The first 102 and second 106 purge valves may direct the fluid being purged substantially out of the system 10' via a muffler 110. The muffler 110 may be configured to reduce noise created by the purging fluid.

The oxygen delivery system 10' may further include a filtration system 114. The filtration system 114 substantially filters particulate matter from the supply fluid and/or the substantially oxygen-rich fluid, depending upon the location of the filtration system 114 within the system 10'. In an embodiment, the filtration system 114 includes a HEPA filter. The system 10' may yet further include a pressure relief valve (as depicted), if desired.

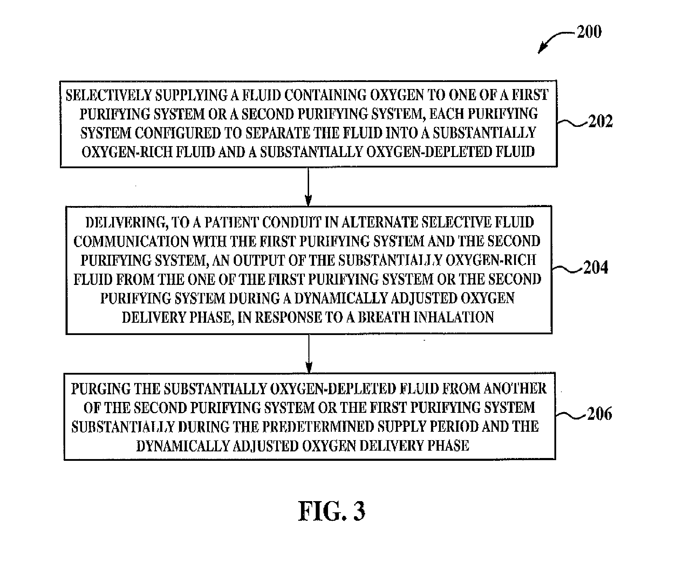

Referring now to Fig. 3, a flow diagram depicting an embodiment of a method 200 for delivering oxygen is illustrated. The embodiment of the method 200 includes selectively supplying a fluid containing oxygen to one of a first purifying system 14 or a second purifying system 18, wherein each purifying system 14, 18 is configured to separate the fluid into a substantially oxygen-rich fluid and a substantially oxygen-depleted fluid, as depicted at reference numeral 202.

The method 200 also includes delivering, to a patient conduit 38 in alternate selective fluid communication with the first purifying system 14 and the second purifying system 18, an output (in response to a breath inhalation) of the substantially oxygen-rich fluid from the first purifying system 14 or the second purifying system 18 during a dynamically adjusted oxygen delivery phase, as depicted at reference numeral 204. The method 200 further includes purging the substantially oxygen-depleted fluid from the other of the second purifying system 18 or the first purifying system 14 substantially during the predetermined supply period and the dynamically adjusted oxygen delivery phase, as depicted at reference numeral 206.

The method 200 further includes selectively supplying, during the predetermined supply period, the supply fluid from the other of the second purifying system 18 or the first purifying system 14, and delivering, to the patient conduit 42, another output of the substantially oxygen-rich fluid from the other of the second purifying system 18 or the first purifying system 14. The substantially oxygen-rich fluid may be output during another dynamically adjusted oxygen delivery phase in response to another breath inhalation.

Further, the method 200 includes purging the substantially oxygen-depleted fluid from the first purifying system 14 or the second purifying system 18 substantially during the other predetermined supply period and the other dynamically adjusted oxygen delivery phase.

Yet further, the method 200 may include transmitting, from the respective purifying system 14, 18, at least a portion of a remaining amount of the substantially oxygen-rich fluid to the other respective purifying system 18, 14. The remaining amount may be transmitted after the respective dynamically adjusted oxygen delivery phase.

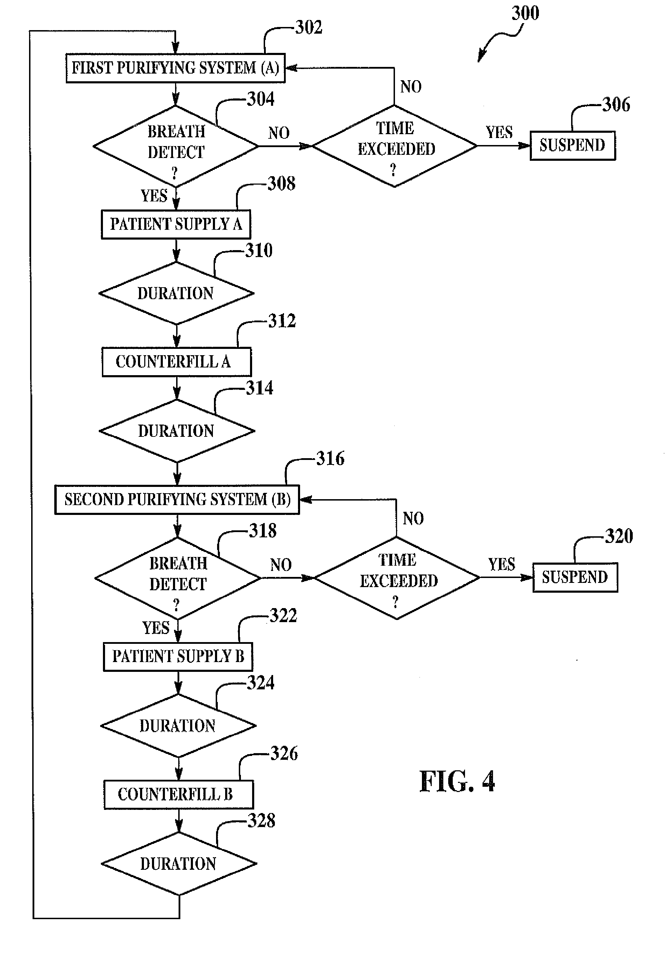

An example embodiment of a method 300 of delivering a substantially oxygen-rich fluid from an oxygen delivery system 10 having first (A) 14 and second (B) 18 purifying systems is depicted in the state diagram of Fig. 4. In the method 300, a supply fluid is supplied to the first purifying system (A) 14 and separated into a substantially oxygen-rich fluid and a substantially oxygen-depleted fluid, as depicted at reference numeral 302.

The system 10 monitors for an inhalation, as depicted at reference numeral 304. If an inhalation is not detected within the predetermined detection time, the system 10 enters suspend mode, as depicted at reference numeral 306. If an inhalation is detected within the predetermined detection time, an output of the substantially oxygen-rich fluid is selectively delivered from the first purifying system (A) 14 to a patient conduit 38, as depicted at reference numeral 308. The output is delivered during a dynamically adjusted oxygen delivery phase, as depicted at reference numeral 310.

While the first purifying system (A) 14 receives and separates the supply fluid, and delivers the substantially oxygen-rich fluid, the second purifying system (B) 18 is configured to substantially purge the substantially oxygen-depleted fluid therein. Next, a predetermined remaining amount of the substantially oxygen-rich fluid is transmitted (counterfilled) from the first purifying system (A) 14 to the second purifying system (B) 18, as depicted at reference numeral 312. The duration of the counterfill may be dynamically calculated (as described hereinabove), as depicted at reference numeral 314.

Next, the supply fluid is supplied to the second purifying system (B) 18 and separated into a substantially oxygen-rich fluid and a substantially oxygen-depleted fluid, as depicted at reference numeral 316. The system 10 monitors for another inhalation, as depicted at reference numeral 318. If an inhalation is not detected within the predetermined detection time, the system 10 enters suspend mode, as depicted at reference numeral 320. If an inhalation is detected within the predetermined detection time, an output of the substantially oxygen-rich fluid is selectively delivered from the second purifying system (B) to the patient conduit 38, as depicted at reference numeral 322. The output is delivered during a dynamically adjusted oxygen delivery phase, as depicted at reference numeral 324.

While the second purifying system 18 receives and separates the supply fluid, and delivers the substantially oxygen-rich fluid, the first purifying system 14 is configured to substantially purge the substantially oxygen-depleted fluid remaining therein. Next, at least a portion of a remaining amount of the substantially oxygen-rich fluid is transmitted (counterfilled) from the second purifying system (B) 18 to the first purifying system (A) 14, as depicted at reference numeral 326. The duration of the counterfill may be dynamically calculated (as described hereinabove), as depicted at reference numeral 328. The method 300 repeats, as desired.

It is to be understood that the terms connect/connected/connection" and/or the like are broadly defined herein to encompass a variety of divergent connected arrangements and assembly techniques. These arrangements and techniques include, but are not limited to (1) the direct communication between one component and another component with no intervening components therebetween; and (2) the communication of one component and another component with one or more components therebetween, provided that the one component being "connected to" the other component is somehow in operative communication with the other component (notwithstanding the presence of one or more additional components therebetween). Additionally, two components may be permanently, semi-permanently, or releasably engaged with and/or connected to one another.

While several embodiments have been described in detail, it will be apparent to those skilled in the art that the disclosed embodiments may be modified. Therefore, the foregoing description is to be considered exemplary rather than limiting.

## Claims

1. An oxygen delivery system, comprising:
a supply fluid containing oxygen;
a first sieve bed and a second sieve bed, each in selective fluid communication with the supply fluid, each of the first and second sieve beds configured to selectively receive the supply fluid during a predetermined supply period, and each further configured to separate a substantially oxygen-rich fluid from the supply fluid;
a patient conduit in alternate selective fluid communication with the first and second sieve beds, the patient conduit having a patient outlet;
a breath-detection device in fluid communication with the patient conduit and configured to trigger an output, having a target flow rate and a flow rate, of a predetermined volume of the substantially oxygen-rich fluid alternately from a respective one of the first sieve bed or the second sieve bed via the patient outlet during a respective dynamically adjusted patient oxygen delivery phase in response to a breath inhalation, the breath detection device further configured to calculate a rate of breathing; and
a device in fluid communication with the patient conduit and configured to measure the output flow rate at predetermined intervals;
wherein each of the respective dynamically adjusted patient oxygen delivery phases is dependent upon at least one of the output target flow rate, the output flow rate, the breathing rate, or combinations thereof.

2. The oxygen delivery system of claim 1 wherein the respective one of the first sieve bed or the second sieve bed is configured to transmit, after the respective dynamically adjusted oxygen delivery phase, at least a portion of a remaining amount of the substantially oxygen-rich fluid to a respective other of the second sieve bed or the first sieve bed.

3. The oxygen delivery system of claim 2 wherein each of the first and second sieve beds is configured to adsorb a substantially oxygen-depleted fluid from the supply fluid during the separating; and
wherein each of the first and second sieve beds is configured to purge the oxygen-depleted fluid substantially after transmitting the at least a portion of the remaining amount of the substantially oxygen-rich fluid to the respective other of the second or first sieve bed.

4. The oxygen delivery system of claim 1 wherein the breath-detection device includes a pressure sensor configured to monitor a pressure of the substantially oxygen-rich fluid in the patient conduit substantially near the patient outlet and to associate a predetermined drop in the pressure with the breath inhalation.

5. The oxygen delivery system of claim 1, further comprising an alert system configured to emit an alarm if detection of the breath inhalation fails to occur within a predetermined amount of time.

6. The oxygen delivery system of claim 5 wherein the predetermined amount of time ranges from about 15 seconds to about 30 seconds.

7. The oxygen delivery system of claim 1, further comprising a filtration system configured to substantially filter particulate matter from at least one of the supply fluid or the substantially oxygen-rich fluid.

8. A method for delivering oxygen, comprising:
selectively supplying, during a predetermined supply period, a fluid containing oxygen to one of a first purifying system or a second purifying system, each of the purifying systems configured to separate the fluid into a substantially oxygen-rich fluid and a substantially oxygen-depleted fluid;
delivering, to a patient conduit in alternate selective fluid communication with the first purifying system and the second purifying system, an output of the substantially oxygen-rich fluid from the one of the first purifying system or the second purifying system during a dynamically adjusted oxygen delivery phase, in response to a breath inhalation, the output having a target flow rate and a flow rate; and
purging the substantially oxygen-depleted fluid from an other of the second purifying system or the first purifying system substantially during the predetermined supply period and the dynamically adjusted oxygen delivery phase.

9. The method of claim 8, further comprising masking detection of the breath inhalation during the dynamically adjusted oxygen delivery phase and during a predetermined amount of time following the delivery phase.

10. The method of claim 8, further comprising transmitting, from the one of the first purifying system or the second purifying system, at least a portion of a remaining amount of the substantially oxygen-rich fluid to the other of the second purifying system or the first purifying system after the dynamically adjusted oxygen delivery phase.

11. The method of claim 8, further comprising:
monitoring a pressure of the substantially oxygen-rich fluid in the patient conduit; and
associating a predetermined drop in the pressure with the breath inhalation.

12. The method of claim 11, further comprising:
resolving a breathing rate based upon a sequence of a plurality of the breath inhalations; and
adjusting the dynamically adjusted patient oxygen delivery phase based upon at least one of the output target flow rate, the output flow rate, the breathing rate, or combinations thereof.

13. The method of claim 11, further comprising emitting an alarm if breath inhalation fails to occur within a predetermined amount of time.

14. The method of claim 13 wherein the predetermined amount of time ranges from about 15 seconds to about 30 seconds.

15. The method of claim 8, further comprising:
selectively supplying, during an other predetermined supply period, the fluid to the other of the second purifying system or the first purifying system;
delivering, to the patient conduit, an output of the substantially oxygen-rich fluid from the other of the second purifying system or the first purifying system during an other dynamically adjusted oxygen delivery phase, in response to an other breath inhalation; and
purging the substantially oxygen-depleted fluid from the one of the first purifying system or the second purifying system substantially during the other predetermined supply period and the other dynamically adjusted oxygen delivery phase.

16. The method of claim 15, further comprising transmitting, from the other of the second purifying system or the first purifying system, at least a portion of a remaining amount of the substantially oxygen-rich fluid to the one of the first purifying system or the second purifying system after the other dynamically adjusted oxygen delivery phase.

17. The method of claim 8 wherein separating the fluid includes substantially adsorbing the oxygen-depleted fluid from the supply fluid.

18. The method of claim 8, further comprising substantially filtering particulate matter from at least one of the supply fluid or the substantially oxygen-rich fluid before delivering the output.

19. The method of claim 12 wherein adjusting the dynamically adjusted patient oxygen delivery phase is based upon each of the output target flow rate, the output flow rate, and the breathing rate.
